(19) Europäisches Patentamt
European Patent Office
Office européen des brevets

(11) Veröffentlichungsnummer: **0 429 999 A1**

# (12) EUROPÄISCHE PATENTANMELDUNG

(21) Anmeldenummer: **90121995.6**

(22) Anmeldetag: **17.11.90**

(51) Int. Cl.[5]: **C07D 519/00**, A61K 31/545

(30) Priorität: **30.11.89 DE 3939533**

(43) Veröffentlichungstag der Anmeldung: **05.06.91 Patentblatt 91/23**

(84) Benannte Vertragsstaaten: **AT BE CH DE DK ES FR GB GR IT LI LU NL SE Patentblatt**

(71) Anmelder: **BAYER AG**

**W-5090 Leverkusen 1 Bayerwerk(DE)**

(72) Erfinder: **Schneider, Stephan, Dr.**
**Claudiusweg 3**
**W-5600 Wuppertal 1(DE)**
Erfinder: **Endermann, Rainer, Dr.**
**In den Birken 152a**
**W-5600 Wuppertal 1(DE)**
Erfinder: **Haller, Ingo, Dr.**
**Dornröschenweg 4**
**W-5600 Wuppertal 1(DE)**
Erfinder: **Metzger, Karl Georg, Dr.**
**Pahlkestrasse 75**
**W-5600 Wuppertal 1(DE)**

## (54) Neue Cephalosporine, Verfahren zu ihrer Herstellung und ihre Verwendung als Arzneimittel.

(57) Die Erfindung betrifft β-Lactam-verbindungen der allgemeinen Formel

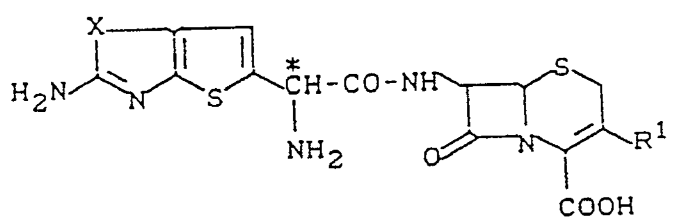

in welcher $R^1$, $R^2$, $R^3$ und X die in der Beschreibung angegebene Bedeutung haben und deren physiologisch unbedenklichen Salze.

EP 0 429 999 A1

# NEUE CEPHALOSPORINE, VERFAHREN ZU IHRER HERSTELLUNG UND IHRE VERWENDUNG ALS ARZNEIMITTEL

Die Erfindung betrifft B-Lactam-Verbindungen der allgemeinen Formel (1),

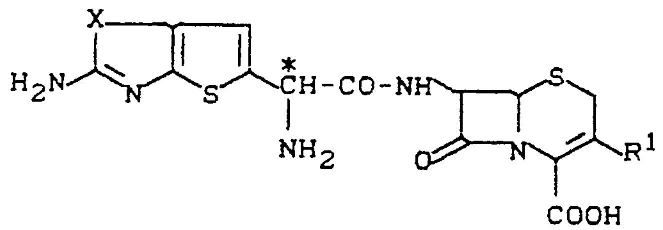

(I)

in welcher

$R^1$ - für Halogen oder
- für geradkettiges oder verzweigtes Alkyl, Alkylthio oder Alkenyl mit bis zu 10 Kohlenstoffatomen steht, die gegebenenfalls durch Halogen, geradkettiges oder verzweigtes Alkoxy mit bis zu 8 Kohlenstoffatomen oder durch einen 5-gliedrigen ungesättigten Heterocyclus mit bis zu 4 Heteroatomen aus der Reihe Stickstoff oder Schwefel substituiert sind, der seinerseits durch geradkettiges oder verzweigtes Alkyl oder Alkylaminodialkyl mit bis zu 6 Kohlenstoffatomen substituiert sein kann, oder die gegebenenfalls durch eine Gruppe der Formel $-0C0R^2$ substituiert sind, worin

$R^2$ - Amino oder geradkettiges oder verzweigtes Alkyl mit bis zu 6 Kohlenstoffatomen bedeutet,
- für geradkettiges oder verzweigtes Alkoxy mit bis zu 8 Kohlenstoffatomen steht,
- für eine Gruppe der Formel $-CH_2-S-R^3$ steht,
worin

$R^3$ - einen 5-gliedrigen ungesättigten Heterocyclus mit bis zu 4 Heteroatomen aus der Reihe Stickstoff oder Schwefel bedeutet, der gegebenenfalls durch geradkettiges oder verzweigtes Alkyl oder Alkylaminodialkyl mit bis zu 6 Kohlenstoffatomen substituiert ist,

X - für Sauerstoff, Schwefel oder für den Rest -NH steht
und deren physiologisch unbedenklichen Salze.

Die Verbindungen der Formel (I) können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium- oder Ammoniumsalze, mit Aminen wie Di-, Tri-niedrigalkylaminen, Procain, Dibenzylamin, N,N'-Dibenzylethylendiamin, N-Benzyl-B-phenyl-ethylamin, N-Methyl-und NEthylmorpholin, 1-Ephenamin, Dihydroabiethylamin, N,N'Bis-dihydroabiethylethylendiamin, N-Niedrigalkylpiperidin und anderen Aminen, die zur Bildung von Salzen von Penicillinen und Cephalosporinen verwendet werden können, vorliegen.

Als nicht-toxische, pharmazeutisch verträgliche Salze der basischen Aminogruppen mit anorganischen oder organischen Säureresten sind beispielsweise Chlorid, Bromid, Iodid, Sulfat, Hydrogensulfat, Phosphat, Carbonat, Hydrogencarbonat, oder Sulfonate wie Methylsulfonat, Ethansulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat oder Lactat bevorzugt zu nennen.

Wegen der Anwesenheit des mit * bezeichneten asymmetrischen Kohlenstoffatoms schließen die neuen ß-Lactamantibiotika der Formel (I) die D-, L- und D,L-Form ein. Bevorzugt sind die D-Formen der erfindungsgemäßen Verbindungen der allgemeinen Formel (I).

Sowohl die Diastereomerengemische als auch die D-Form und L-Form der erfindungsgemäßen Verbindungen können zur Behandlung bakterieller Infektionskrankheiten eingesetzt werden.

Bevorzugt sind Verbindungen der allgemeinen Formel (I),
in welcher

$R^1$ - für Fluor, Chlor oder Brom steht, oder
- für geradkettiges oder verzweigtes Alkyl, Alkylthio oder Alkenyl mit bis zu 8 Kohlenstoffatomen steht, die gegebenenfalls durch Fluor, Chlor, Brom oder durch geradkettiges oder verzweigtes Alkoxy mit bis zu 6 Kohlenstoffatomen oder durch Tetrazolyl, Thiazolyl, Triazolyl oder Thiadiazolyl substituiert sind, welche ihrerseits durch geradkettiges oder

verzweigtes Alkyl oder Alkylaminodialkyl mit bis zu 4 Kohlenstoffatomen substituiert sein können, oder

- die gegebenenfalls durch eine Gruppe der Formel -0C0R² substituiert sind, worin

$R^2$ - Amino oder geradkettiges oder verzweigtes Alkyl mit bis zu 4 Kohlenstoffatomen bedeutet,

- für geradkettiges oder verzweigtes Alkoxy mit bis zu 6 Kohlenstoffatomen steht,

X - für Sauerstoff oder Schwefel steht,
und deren physiologisch unbedenklichen Salze.
Besonders bevorzugt sind Verbindungen der allgemeinen Formel (I),
in welcher

$R^1$ - für Chlor oder geradkettiges oder verzweigtes Alkyl oder Alkenyl mit bis zu 6 Kohlenstoffatomen steht, die gegebenenfalls durch Fluor, Chlor oder geradkettiges oder verzweigtes Alkoxy mit bis zu 4 Kohlenstoffatomen substituiert sind, oder

- für geradkettiges oder verzweigtes Alkoxy mit bis zu 4 Kohlenstoffatomen steht,

x - für Schwefel steht
und deren physiologisch unbedenklichen Salze.

Außerdem wurde ein Verfahren zur Herstellung der Verbindungen der allgemeinen Formel (I),

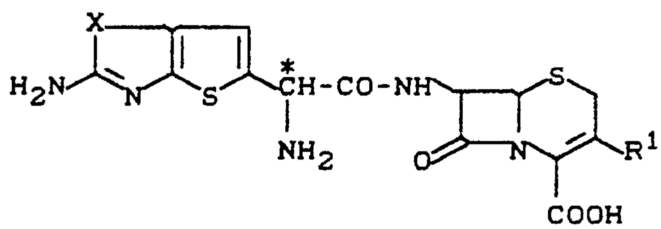

(I)

in welcher
$R^1$ und X die oben angegebene Bedeutung haben,
gefunden, dadurch gekennzeichnet, daß man entweder

[A] in einer 4-Komponentenreaktion Verbindungen der allgemeinen Formeln (II), (III) und (IV),

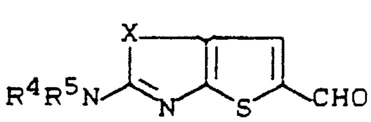

(II) ,

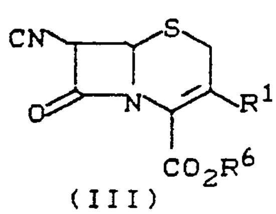

(III)

und

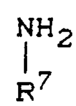

(IV)

in welchen

$R^1$ und X die oben angegebene Bedeutung haben,

$R^4$ und $R^5$ gleich oder verschieden sind und für eine Aminoschutzgruppe, beispielsweise für tert.-Butyloxycarbonyl (Boc) stehen,

$R^6$ für eine Carboxyschutzgruppe steht,
und

$R^7$ - für einen leicht abspaltbaren, benzylisch oder glykosidisch gebundenen Rest, wie beispielsweise

4,4'-Dimethoxybenzhydryl oder 2,4-Dimethoxybenzyl oder Tetra-O-pivaloylgalactosyl steht, oder

- für einen benzylisch, chiralen Rest, wie beispielsweise ∝-Ferrocenyl-($C_2$-$C_6$)-alkylamino steht,

in Anwesenheit einer Carbonsäure der Formel (V),

$$R^8\text{-COOH} \quad (V)$$

in welcher

$R^8$ - Wasserstoff, geradkettiges oder verzweigtes Alkyl mit bis zu 6 Kohlenstoffatomen oder einen Rest der Formel

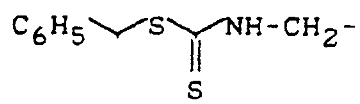

bedeutet,

in inerten Lösemitteln, gegebenenfalls in Anwesenheit eines Katalysators zu Verbindungen der allgemeinen Formel (Ia),

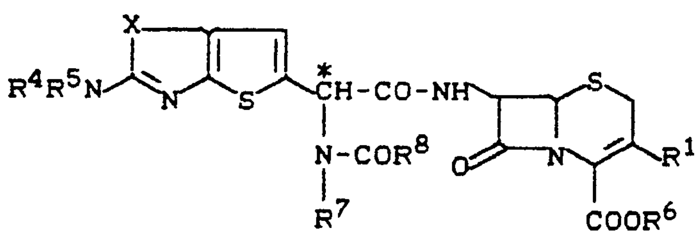

(Ia)

in welcher

$R^1$, $R^4$, $R^5$, $R^6$, $R^7$, $R^8$, und X die oben angegebene Bedeutung haben,

umsetzt, anschließend die Schutzgruppen $R^4$, $R^5$, $R^6$ und die Reste $R^7$ und -$COR^8$ mit Säuren abspaltet,

oder daß man

[B] Carbonsäuren der allgemeinen Formel (VI),

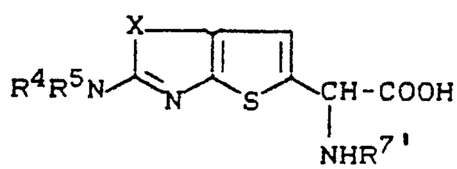

(VI)

in welcher

$R^3$, $R^4$ und X die oben angegebene Bedeutung haben

und

$R^{7'}$ - die oben angegebene Bedeutung von $R^7$ hat und mit dieser gleich oder verschieden ist, oder

- für eine Aminoschutzgruppe steht,

nach Aktivierung der Carboxylgruppe nach üblicher Methode, mit einer ß-Lactam-Verbindung der allgemeinen Formel (VII),

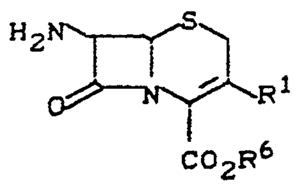

(VII)

in welcher

$R^1$ und $R^6$ die oben angegebene Bedeutung haben,

in inerten Lösemitteln umsetzt und im letzten Schritt gegebenenfalls die Schutzgruppen $R^4$, $R^5$, $R^6$ und $R^{7'}$ abspaltet.

Die erfindungsgemäßen Verfahrensvarianten können durch folgende Formelschemata erläutert werden:

[A]

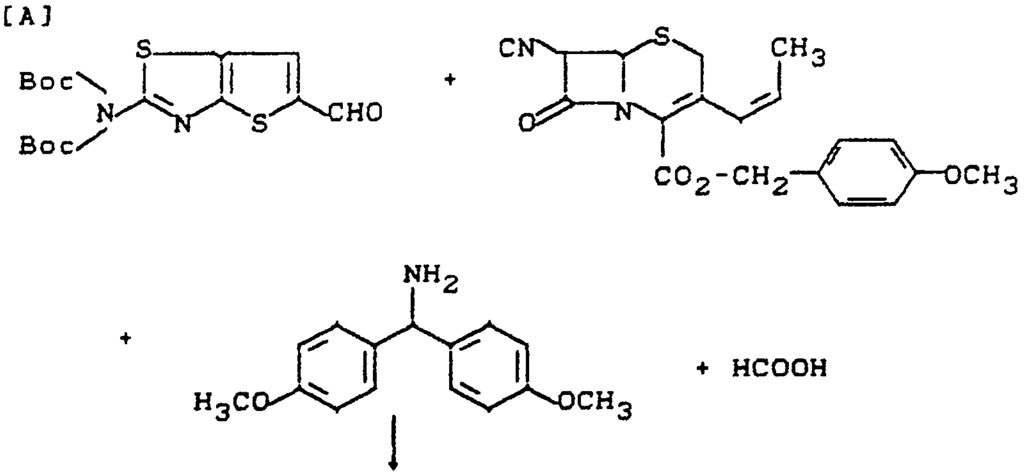

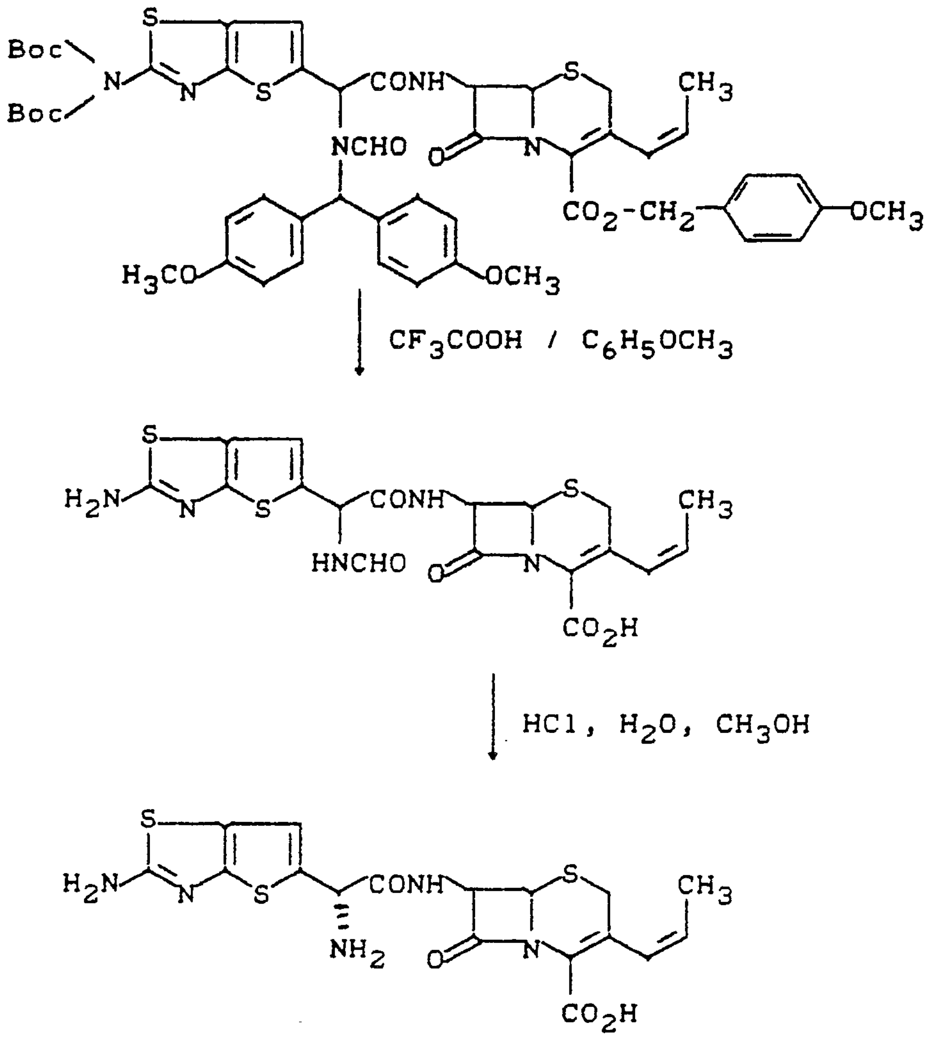


[B]

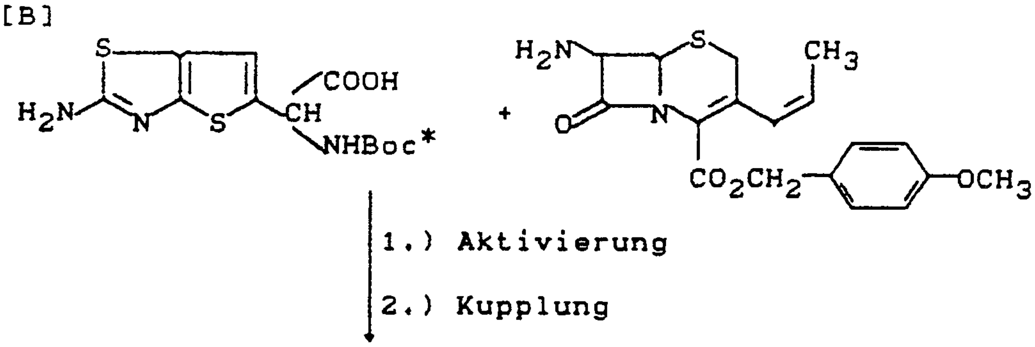

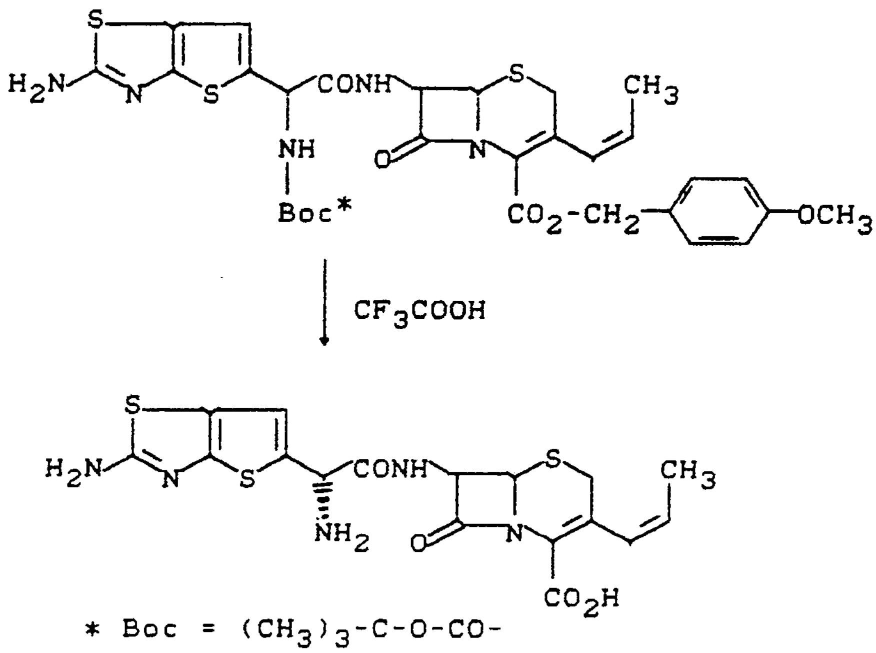


* Boc = $(CH_3)_3$-C-O-CO-

Aminoschutzgruppe im Rahmen der oben angegebenen Definition steht im allgemeinen für eine in der ß-Lactam-Chemie üblichen Schutzgruppe aus der Reihe: 4-Methoxy-phenyl, 4-Methoxymethyloxyphenyl, 4-[(2-Methoxyethoxy)-methyloxy]phenyl, 3,4-Dimethoxyphenyl, Benzyl, 2-Nitro-benzyl, 4-Nitrobenzyl, 4-Methoxybenzyl, 2,4-Dimethoxy-benzyl, 3,4-Dimethoxybenzyl, 2,4,6-Trimethoxybenzyl, Vinyl, Allyl, tert-Butoxycarbonyl, Benzyloxycarbonyl, 2-Nitrobenzyloxycarboyl, 4-Nitrobenzyloxycarbonyl, Formyl, Acetyl, Chloracetyl, Trichloracetyl, Trifluoracetyl, Benzoyl, Methoxycarbonyl, Allyloxycarbonyl, 2,4-Dimethoxybenzyloxycarbonyl, 2,2-Diethoxyethyl, Methoxycarbonylmethyl, tert-Butoxycarbonylmethyl, Allyloxymethyl, Benzoylmethyl, Bis-(4-methoxyphenyl)methyl, Methoxymethyl, Methylthiomethyl, Methoxyethoxymethyl, 2-(Methylthiomethoxy)ethoxycarbonyl, 2-Hydroxy-2-phenyl-methyl, Methoxy-(4-methoxyphenyl)methyl, Trimethyl-, Triethyl-, Triphenylsilyl, tert-Butyl-dimethylsilyl, tert-Butyl-diphenylsilyl, [2-(Trimethylsilyl)ethoxyl-methyl, 1-Methyl-2-benzyoyl-vinyl, 1-Methyl-2-ethoxy- oder 2-methoxyvinyl, Mesyl- und Ethylsulfonyl.

Carboxyschutzgruppe im Rahmen der oben angegebenen Definition steht für die in der ß-Lactam-Chemie üblichen Carboxyschutzgruppe. Bevorzugt sind leicht abspaltbare Gruppen zu nennen, wie zum Beispiel: tert.Butyl, 2,2,2-Trichlorethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-pro-penyl.

Als Lösemittel für die Verfahrensvariante [A] (4-Kom-ponentenreaktion) eignen sich die üblichen organischen Lösemittel, die sich unter den Reaktionsbedingungen nicht verändern. Hierzu gehören bevorzugt Ether wie Diethylether, Butylmethylether, Dioxan oder Tetrahydrofuran, oder Kohlenwasserstoffe wie Benzol, Toluol, Xylol, oder Cyclohexan, oder Amide wie Dimethylformamid oder Hexamethylphosphorsäuretriamid, oder Alkohole wie Methanol, Ethanol, Propanol oder Isopropanol, oder Chlorkohlenwasserstoffe wie Methylenchlorid, Chloroform oder Tetrachlorkohlenstoff, oder Aceton, Acetonitril oder Essigester. Ebenso ist es möglich, Gemische der genannten Lösemittel zu verwenden. Bevorzugt ist Methanol.

Die Umsetzung kann in einem Temperaturbereich von 0° C bis +50° C, vorzugsweise bei Raumtemperatur durchgeführt werden.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Die Carbonsäuren der allgemeinen Formel (V) sind bekannt. Bevorzugt werden Ameisensäure oder N-Dithiocarbobenzyloxyglycin eingesetzt.

Bei der 4-Komponentenreaktion wird die Carbonsäure in einer Menge von 1 bis 20 Mol, bevorzugt mit 10 Mol, bezogen auf jeweils 1 Mol der Verbindungen der allgemeinen Formeln (II), (III) und (IV) eingesetzt.

Für den Fall, daß $R^7$ in der Formel (IV) für einen glykosidisch gebundenen Rest steht, ist der Einsatz von Katalysatoren bei der 4-Komponentenreaktion [A] notwendig. Als Katalysatoren eignen sich Zinksalze und Titankomplexe, wie beispielsweise Zinkchlorid und Ti(4)-iso-propylat. Der Katalysator wird in einer Menge von 0,5 bis 5 Mol, bevorzugt mit 1 Mol, bezogen auf 1 Mol der Verbindungen der Formel (IV),

eingesetzt.

Die Abspaltung der Amin- und Carboxyschutzgruppen erfolgt bei den Verfahren [A] und [B] bevorzugt mit Tri fluoressigsäure. Es ist aber auch möglich andere, literaturbekannte Methoden anzuwenden.

Die Verbindungen der allgemeinen Formel (Ia) sind ebenfalls neu.

Die sich anschließende Abspaltung der Formyl- oder Acylgruppe aus den Verbindungen der allgemeinen Formel (Ia) erfolgt ebenfalls mit Trifluoressigsäure, wenn $R^8$ nicht Wasserstoff bedeutet. Für den Fall, daß $R^8$ für Wasserstoff steht, werden Protonensäuren, vorzugsweise wäßrige Salzsäure in einem Methanol/Wasser-Gemisch, eingesetzt.

Die Abspaltung des Restes $R^7$ (Verbindungen der Formel Ia) wird durch Zugabe von Hilfsstoffen, wie beispielsweise Anisol, Phenol, Thiophenol oder Phenylthioether, bevorzugt Anisol, erleichert.

Die Abspaltungen der Schutzgruppen und der Acyl/Formylgruppe werden im allgemeinen in einem Temperaturbereich von 0° C bis +80° C, vorzugsweise bei Raumtemperatur durchgeführt.

Die Abspaltungen können sowohl bei Normaldruck als auch bei erhöhtem oder erniedrigtem Druck (z.B. 0,5 bis 5 bar), vorzugsweise bei Normaldruck, durchgeführt werden.

Als Lösemittel für die Verfahrensvariante [B] eignen sich alle Solventien, die sich unter den Reaktions bedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen. Bevorzugt ist Methylenchlorid.

Die Kondensation erfolgt im allgemeinen in einem Temperaturbereich von 0° C bis +60° C, vorzugsweise bei Raumtemperatur und bei Normaldruck.

Die Abspaltung der Schutzgruppen erfolgt nach der unter [A] beschriebenen Methode.

Die Aktivierung der Carboxylgruppe in den Verbindungen der allgemeinen Formel (VI) erfolgt im allgemeinen durch Überführen in ein gemischtes Anhydrid mit Chlorameisensäureestern oder Sulfonsäurederivaten, wie beispielsweise Chlorameisensäureethylester, Chlorameisensäureisobutylester oder Methansulfonsäurechlorid, durch Überführen in das entsprechende Säurehalogenid, oder durch Überführen in einen aktivierten Ester, beispielsweise mit N-Hydroxybenztriazol und Dicyclohexylcarbodiimid. Bevorzugt ist die Umsetzung mit Chlorameisensäureethylester und Methansulfonsäurechlorid.

Als Lösemittel für die Einführung der Aminoschutzgruppen bei den Verfahrensvarianten [A] und [B] eignen sich alle Solventien, die sich unter den Reaktionsbedingungen nicht verändern. Hierzu gehören bevorzugt Chlorkohlenwasserstoffe wie Methylenchlorid, Chloroform oder Tetrachlorkohlenstoff, oder Amide wie Dimethylformamid oder Hexamethylphosphorsäuretriamid, oder Acetonitril, Ameisensäure, Aceton, Pyridin oder Dimethylsulfoxid. Bevorzugt sind Pyridin, Dimethylsulfoxid und Dimethylformamid.

Die Verbindungen der allgemeinen Formel (II) sind neu und können hergestellt werden, indem man Verbindungen der allgemeinen Formel (VIII),

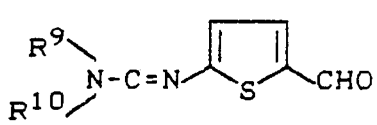

(VIII)

in welcher

$R^9$ und $R^{10}$ gleich oder verschieden sind und für geradkettiges oder verzweigtes Alkyl mit bis zu 6 Kohlenstoffatomen oder Phenyl stehen,

nach üblicher Methode, beispielsweise durch Rhodanierung, Nitrierung und anschließender Reduktion oder Formylierung mit anschließender Oxidation in inerten Lösemitteln zu Verbindungen der allgemeinen Formel (IX)

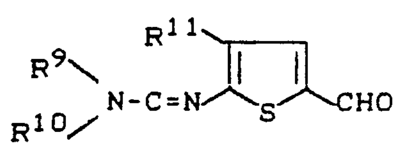

(IX)

in welcher

$R^{11}$ beispielsweise für die Gruppe $-NH_2$,

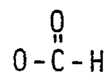

oder -SCN steht

und

$R^9$ und $R^{10}$ die oben angegebene Bedeutung haben,

umsetzt,

und anschließend in schwach saurem, wäßrigem Medium, wie beispielsweise mit Br-CN oder in Essigsäure/Wasser zu Verbindungen der allgemeinen Formel (IIa),

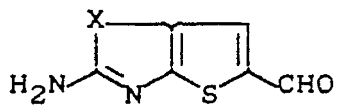

(IIa)

in welcher

X die oben angegebene Bedeutung hat,

cyclisiert, und in einem letzten Schritt die Aminfunktion durch Einführung der Schutzgruppen ($R^4/R^5$) blockiert.

Die Einführung des Restes $R^{11}$ erfolgt nach literaturbekannten Verfahren,

Als Lösemittel für die Einführung des Restes $R^{11}$ eignen sich die unter Verfahren [A] aufgeführten Solventien. Bevorzugt sind Alkohole oder Chlorkohlenwasserstoffe, wie beispielsweise Methanol, Ethanol , Propanol , Isopropanol oder Methylenchlorid. Die Reaktion verlauft in einem Temperaturbereich von 0° C bis +80° C, vorzugsweise bei Raumtemperatur und bei Normaldruck.

Die Cyclisierung wird in einem Temperaturbereich von 40° C bis 150° C, bevorzugt bei 50° C bis 80° C und bei Normaldruck durchgeführt.

Die Verbindungen der allgemeinen Formel (VI) sind neu und können hergestellt werden, indem man Verbindungen der allgemeinen Formel (II),

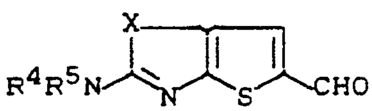

(II),

in welcher

$R^4$, $R^5$ und X die oben angegebene Bedeutung haben,

mit Alkalicyaniden, wie beispielsweise Kaliumcyanid, oder Trimethylsilylcyanid und Aminen der Formel (IV),

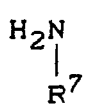

(IV)

in welcher

$R^7$ die oben angegebene Bedeutung hat,

oder mit Ammoniumacetat oder Ammoniak zu Verbindungen der allgemeinen Formel (X),

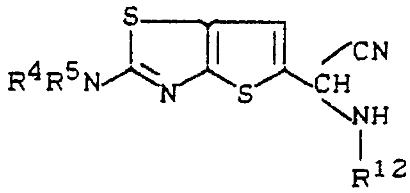

(X)

in welcher
$R^4$ und $R^5$ die oben angegebene Bedeutung haben,
und

$R^{12}$ - den oben angegebenen Bedeutungsumfang von $R^7$ hat und im Fall von Ammoniak auch Wasserstoff bedeutet

umsetzt, und anschließend mit Säuren, beispielsweise Salzsäure, die Schutzgruppen $R^4$, $R^5$ und $R^{12}$ ($R^{12} \neq$ H) abspaltet und Cyanogruppen hydrolysiert und gegebenenfalls eine andere der oben aufgeführten Aminoschutzgruppen einführt.

Die Umsetzung mit den Cyaniden verläuft im allgemeinen in einem Temperaturbereich von 0 °C bis +50 °C, vorzugsweise bei Raumtemperatur und Normaldruck.

Die Abspaltung der Aminschutzgruppen erfolgt nach der oben aufgeführten Methode in einem Temperaturbereich von +60 °C bis +150 °C, vorzugsweise bei +70 °C bis +90 °C bei Normaldruck.

Die Verbindungen der allgemeinen Formeln (VIII) und (IX) sind an sich bekannt oder können nach an sich bekannten Verfahren hergestellt werden.

Die Verbindungen der allgemeinen Formel (VII) sind an sich bekannt oder können nach üblicher Methode hergestellt werden [vgl. DE-OS 34 02 642, US-Pat. 4 639 448].

Die Verbindungen der allgemeinen Formel (III) sind teilweise bekannt oder neu und können in beiden Fällen in Analogie zu literaturbekannten Verfahren hergestellt werden [vgl. DOS 23 37 107].

Die Amine der allgemeinen Formel (IV) sind an sich bekannt oder können nach üblicher Methode hergestellt werden.

Die stereochemisch einheitliche D- bzw. L-Form der erfindungsgemäßen Verbindungen der Formel (I) erhält man, wenn man die Diastereomerengemische durch präparative HPLC-Trennmethoden isoliert.

Andererseits erhält man die reine D- bzw. L-Form (bevorzugt die D-Form), wenn man bereits auf der Stufe der geschützten oder ungeschützten racemischen Aminosäure der Formel (VI) eine chemische Racematspaltung, z.B. mit Dehydroabiethylamin, Phenylethylamin oder Camphersulfonsäure, oder eine Racematspaltung z.B. über N-Acetyl-aminosäurederivate, z.B. mit Subtilisin, Penicillinacylase oder Schweinenierenacylase, vornimmt und anschließend die stereochemisch einheitlichen D- bzw. L-Formen der Verbindungen der Formel (VI) in angegebener Weise umsetzt, oder indem man auf der Stufe der Formel (X), in welcher $R^{12}$ für einen der oben aufgeführten chiralen Reste steht, direkt in die einzelnen Diastereomere trennt.

Die erfindungsgemäßen Verbindungen der allgemeinen Formel (I) weisen bei geringer Toxizität ein breites antibakterielles Spektrum gegen gram-positive und gramnegative Keime auf. Diese Eigenschaften ermöglichen ihre Verwendung als chemotherapeutische Wirkstoffe in der Human- und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipointlnokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37 °C bebrütet, und das Keimwachstum wurde nach ca. 20 Stunden

abgelesen. Der MHK-Wert (µg/ml) gibt die niedrigste Wirkstoffkonzentration an, bei der mit bloßem Auge kein Keimwachstum zu erkennen war.

Anwendungsbeispiel

MHK-Werte: Agarverdünnung/Multipoint

Verbindung des Beispiels 13a:

| Stamm | MHK (µg/ml) |
|---|---|
| E. coli A 261 | 4 |
| Klebs. 6097 | 2 |
| Staph. aur. 25455 | ≤ 0,25 |
| Staph. epi. 25185 | ≤ 0,25 |
| Strept. faec. 27101 | 16 |
| Enterococc. 27185 | 2 |

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls mit einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeuticche Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

Ausganqsverbindungen

Beispiel 1

5-Dimethylaminomethylenamino-4-thiocyanato-2-thiophen-carboxaldehyd

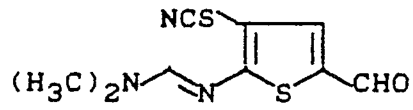

Zu einer Lösung aus 32,0 g (176 mmol) 5-Dimethylamino-methylenamino-2-thiophencarboxaldehyd und 40,1 g Ammoniumthiocyanat (527 mmol) in 2,2 1 Methanol tropft man langsam bei Raumtemperatur 10,0 ml (194 mmol) Brom. Man rührt 1 h bei Raumtemperatur, gibt ca. 2 l Eis/WasserGemisch hinzu, läßt noch 45 Minuten nachrühren, saugt bei 5 °C den entstandenen Niederschlag ab, wäscht mit Wasser und trocknet im Vakuum.

Ausbeute: 24,4 g (58% der Theorie)

$^1$H-NMR (GD3SOCD3): $\delta$ = 3,12 und 3,20 (2s, je 3H, 5'-CH3); 8,00 (s, 1H; 3-H); 8,20 (s, 1H, N-CH = N); 9,63 (s, 1H; CHO).

Beispiel 2

2-Amino-5-thieno[2,3-d]thiazolcarboxaldehyd

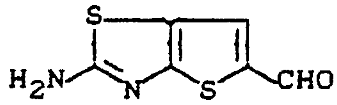

24,4 g (102 mmol) der Verbindung aus Beispiel 1 werden in 170 ml 90%iger Essigsäure aufgeschlämmt und auf 60-65 °C erwärmt, wobei der Aldehyd in Lösung geht. Nach 24 h bei 60-65 °C läßt man auf Raumtemperatur abkühlen. Nach weiteren 24 h saugt man die schwarzen Kristalle ab, wäscht mit Toluol und trocknet im Vakuum.

Ausbeute: 15,4 g (82% der Theorie)

$^1$H-NMR ($CD_3SOCD_3$): $\delta$ = 8,05 (s, 1H, 6-H); 8,22 (s, 2H, $NH_2$); 9,74 (s, 1H, CHO).

Beispiel 3

2-Acetylamino-5-thieno[2,3-d]thiazol-carboxaldehyd

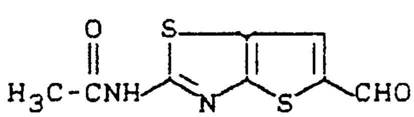

Zu einer Lösung von 15,4 g (84 mmol) der Verbindung aus Beispiel 2 in 660 ml wasserfreiem Pyridin tropft man bei 15-20 °C innerhalb von 1 h eine Lösung von 58,6 ml (824 mmol) Acetylchlorid in 660 ml wasserfreiem Chloroform. Nach 30 Minuten gießt man auf eine Mischung aus 1 1 Wasser und 1 1 Chloroform, trennt die Phasen und wäscht die organische Phase mehrfach mit Wasser. Die organische Phase wird über eine wenige Zentimeter dicke Schicht Kieselgel filtriert und anschließend im Vakuum eingeengt. Der so erhaltene Rückstand wird mehrfach mit Toluol abgezogen, bis er kristallisiert. Die rotbraunen Kristalle werden über Nacht mit 450 ml Methanol verrührt, abgesaugt, mit Methanol gewaschen und im Vakuum getrocknet.

Ausbeute: 11,2 g (59% der Theorie)

$^1$H-NMR ($CD_3SOCD_3$): $\delta$ = 2,23 (s, 3H, $CH_3CO$); 8,30 (s, 1H, 6-H); 9,91 (s, 1H, CHO); 12,65 (s, 1H, NH).

Beispiel 4

2-Formylamino-5-thieno[2,3-d]thiazol-carboxaldehyd

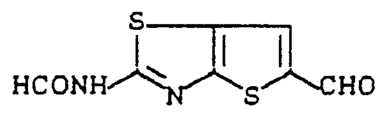

1,84 g (10 mmol) der Verbindung aus Beispiel 2 in 100 ml Ameisensäure werden bei 15-20° C tropfenweise mit 20 ml (212 mmol) Acetanhydrid versetzt. Man rührt über Nacht bei Raumtemperatur, versetzt anschließend mit 500 ml Eiswasser und saugt den ausgefallenen Niederschlag ab. Die Kristalle werden mit Essigester verrührt, abgesaugt und im Vakuum getrocknet.
Ausbeute: 1,10 g (52% der Theorie)
$^1$H-NMR ($CD_3SOCD_3$): $\delta$ = 8,33 (s, 1H, 6-H); 8,64 (s, 1H, N-CHO); 9,92 (s, 1H, C-CHO); 12,85 (s, 1H, NH).

Beispiel 5

2-(N,N-Di-t-butyloxycarbonyl-amino)-5-thieno[2,3-d]-thiazolcarboxaldehyd

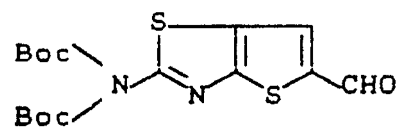

Zu einer Lösung von 1,84 g (10 mmol) der Verbindung aus Beispiel 2 gibt man bei Raumtemperatur 21,8 g (100 mmol) Pyrokohlensäure-di-t-butylester und rührt anschließend über Nacht bei derselben Temperatur. Man versetzt tropfenweise mit 3 ml Wasser, engt im Vakuum ein und zieht den Rückstand mehrfach mit Toluol ab. Chromatographie an Kieselgel mit Toluol/Essigester (97:3) liefert 1,30 g (34% der Theorie) des gewünschten Aldehyds.
$^1$H-NMR ($CD_3SOCD_3$): $\delta$ = 1,55 (s, 18H, Boc); 8,35 (s, 1H, 6-H); 9,96 (s, 1H, CHO).

Beispiel 6

2-Amino-2-(2-amino-5-thieno[2,3-d]thiazolyl )-essigsäure

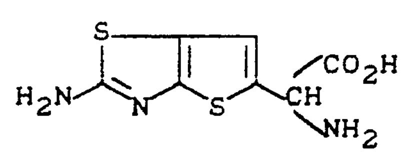

Zu einer Aufschlämmung von 904 mg (4,0 mmol) der Verbindung aus Beispiel 3 in 25 ml Ethanol gibt man 360 mg (5,5 mmol) Kaliumcyanid und 1,23 g (16,0 mmol) Ammoniumacetat und rührt 72 h bei Raumtemperatur. Die Reaktionsmischung wird zur Trockene eingeengt, in 100 ml 6 N Salzsäure aufgenommen und 2 h unter Rückfluß erhitzt. Bei einer Badtemperatur von 50° C wird im Vakuum zur Trockene eingeengt. Den Rückstand löst man in 200 ml Wasser, und anschließend stellt man mit Natriumacetat auf pH 5 ein. Die Suspension wird aufgekocht und heiß filtriert. Das fast farblose Filtrat wird zur Trockene eingeengt und an RP-8 mit Wasser chromatographiert.

Ausbeute: 29,5 mg (3% der Theorie)
$^1$H-NMR (DCOOD): δ = 5,78 (s; 1H, 2-H); 7,58 (s; 1H, 6'-H).

Beispiel 7

(6R, 7R)-7-Formylamino-3-[(Z)-1-propenyl]-3-cephem-4-carbonsäure-p-methoxybenzylester

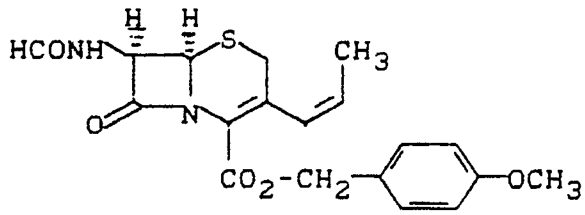

Eine Lösung von 45 g (125 mmol) (6R, 7R)-7-Amino-3-[(Z)-1-propenyl]-3-cephem-4-carbonsäure-p-methoxybenzylester, 9,5 ml (252 mmol) Ameisensäure und 53,8 g Dicyclohexylcarbodiimid (261 mmol) in 750 ml Acetonitril wird über Nacht bei Raumtemperatur gerührt. Man trennt den ausgefallenen Niederschlag ab, engt das Filtrat im Vakuum ein und chromatographiert an Kieselgel mit Toluol/Essigester (3:1).
Ausbeute: 38 g (78% der Theorie)
$^1$H-NMR ($CDC1_3$): δ = 1,57 (dd, J = 6 Hz, J = 1 Hz, 3H, $CH_3$-C); 3,36 und 3,50 (2d, J = 19 Hz, je 1H, 2-H); 3,82 (s, 3H, $CH_3O$); s,02 (d, J = 6 Hz, 1H, 6-H); 5,17 (s, 2H, $CH_2$); 5,68 (dq, J = 11 Hz, J = 6 Hz, 1H, 3'-H); 5,88 (dd, J = 9 Hz, J = 6 Hz, 1H, 7-H); 6,12 (d, J = 11 Hz, 1H, 3'-H); 6,58 (d, J = 9 Hz, 1H, NH); 6,88 und 7,32 (2d, J = 8 Hz, je 2H, Ar-H); 8,28 (s, 1H, NCHO).

Beispiel 8

(6R, 7R)-7-Isocyano-3-[(Z)-1-propenyl]-3-cephem-4-carbonsäure-p-methoxybenzylester

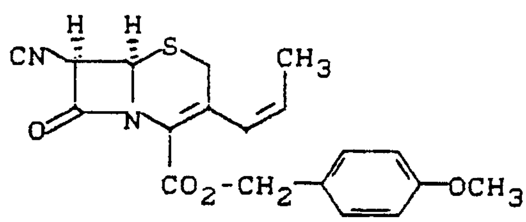

Zu einer Lösung von 7,8 g (20 mmol) der Verbindung aus Beispiel 7 und 6,9 ml (85 mmol) Pyridin in 120 ml wasserfreiem Methylenchlorid tropft man bei -78° C 2,4 ml (20 mmol) Chlorameisensäuretrichlormethylester. Nach 10 Minuten läßt man auf 0° C erwärmen, fügt Eiswasser zu, trennt die organische Phase ab und extrahiert die wäßrige Phase zweimal mit Methylenchlorid. Die vereinigten organischen Phasen werden mit Wasser gewaschen, über Natriumsulfat getrocknet und eingeengt. Chromatographie an Kieselgel mit Methylenchlorid liefert 3,1 g (42% der Theorie) der Titelverbindung.
$^1$H-NMR ($CDC1_3$): δ = 1,63 (dd, J = 6 Hz, J = 1 Hz, 3H, C-$CH_3$); 3,42 und 3,48 (2d, J = 18 Hz, je 1H, 2-H); 3,80 (s, 3H, $OCH_3$); 4,97 (d, J = 6 Hz, 1H, 6-H); 5,17 (m, 3H, 7-H und 4'-$CH_2$); 5,75 (dq, J = 11 Hz, J = 6 Hz, 1H, 3'-H); 6,26 (d, J = 11 Hz, 1H, 3'-H); 6,90 und 7,32 (2d, J = 8 Hz, je 2H, Ar-H).

Beispiel 9

7-{2-[2-(N,N-Di-t-butyloxycarbonyl-amino)-5-thieno[2,3-d]thiazolyl]-2-[N-(4,4'-dimethoxybenzhydryl)-N-formylamino]-acetylamino}-3-[(Z)-1-propenyl]-(6R, 7R)-3-ce-phem-4-carbonsäure-p-methoxybenzylester

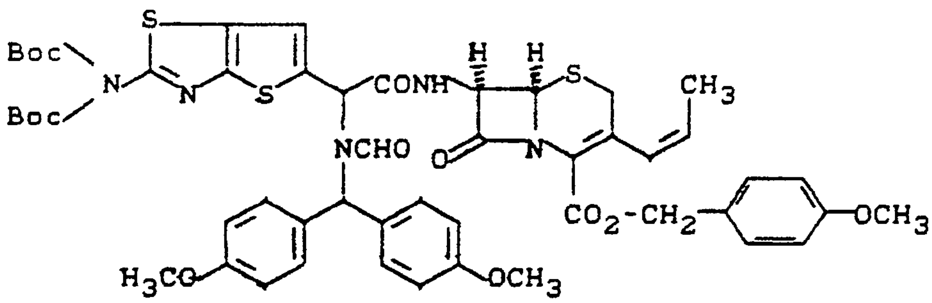

Eine Lösung von 1,30 g (3,5 mmol) der Verbindung aus Beispiel 8, 1,35 g (3,5 mmol) der Verbindung aus Beispiel 5, 0,85 g (3,5 mmol) 4,4'-Dimethoxybenzhydrylamin und 1,3 ml (35 mmol) Ameisensäure in 13 ml Methanol und 1,3 ml Dimethylformamid wird 5 Tage bei Raumtemperatur gerührt. Die Lösung wird eingeengt; der Rückstand wird an Kieselgel mit Toluol/Essigester (8:2) chromatographiert.
Ausbeute: 0,25 g (7% der Theorie)
$R_f$ = 0,31 (Kieselgel 60, F 254, Merck Nr. 5549; Toluol/Essigester 3:1)

Beispiel 10

(6R, 7R)-7-Formylamino-3-[(Z)-1-propenyl]-3-cephem-4-carbonsäure-benzhydrylester

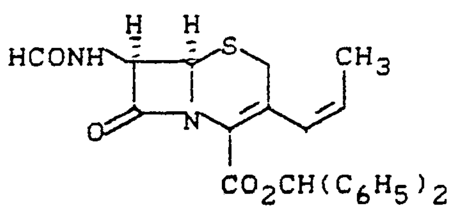

In Analogie zur Vorschrift des Beispiels 7 erhält man aus 8,1 g (20 mmol) (6R, 7R)-7-Amino-3-[(Z)-1-propenyl]-3-cephem-4-carbonsäure-benzhydrylester, 1,5 ml (40 mmol) Ameisensäure und 4,9 g (23,7 mmol) Dicyclohexylcarbodiimid in 70 ml Acetonitril 8,0 g (92% der Theorie) der Titelverbindung.
$^1$H-NMR ($CDC1_3/CD_3SOCD_3$): $\delta$ = 1,44 (dd, J = 6 Hz, J = 1 Hz, 3H, $CH_3$); 3,40 und 3,50 (2d, J = 18 Hz, je 1H, 2-H); 5,08 (d, J = 6 Hz, 1H, 6-H); 5,56 (dq, J = 11 Hz, J = 6 Hz, 1H, 3'-H); 5,83 (dd, J = 9 Hz, J = 6 Hz, 1H, 7-H); 6,12 (d, J = 11 Hz, 1H, 3'-H); 6,92 (s, 1H; $Ph_2CH$); 7,25 - 7,43 (m, 10H, Ph-H); 8,24 (s, 1H, CHO); 8,87 (d, J = 9 Hz, 1H, N-H).

Beispiel 11

(6R, 7R)-7-Isocyano-3-[(Z)-1-propenyll-3-cephem-4-car-bonsäure-benzhydrylester

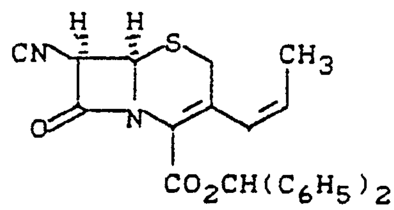

In Analogie zur Vorschrift des Beispiels 8 erhält man aus 4,35 g (10 mmol) der Verbindung aus Beispiel 10, 3,5 ml (43 mmol) Pyridin und 1,2 ml (10 mmol) Chlorameisensäuretrichlormethylester in 60 ml wasserfreiem Methylenchlorid 2,05 8 (49% der Theorie) der Titelverbindung.

$^1$H-NMR ($CDC1_3$): $\delta$ = 1,53 (dd, J = 6 Hz, J = 1 Hz, 3H, $CH_3$); 3,43 und 3,46 (2d, J = 18 Hz, je 1H, 2-H); 5,01 (d, J = 6 Hz, 1H, 6-H); 5,20 (d, J = 6 Hz, 1H, 7-H); 5,69 (dq, J = 11 Hz, J = 6 Hz, 1H, 3'-H); 6,29 (d, J = 11 Hz, 1H, 3 - H); 6,95 (s, 1H; $CHPh_2$); 7,28 - 7,42 (m, 10H, Ph-H).

Beispiel 12

7-{2-[2-(N,N-Di-t-butyloxycarbonylamino)-5-thieno[2,3-d]thiazolyl]-2-[N-(tetra-0-pivaloyl-1-galactosyl)-N-formyl-amino]-(2S)-acetylamino}-3-[(Z)-1-propenyl]-(6R, 7R)-3-cephem-4-carbonsäure-benzhydrylester

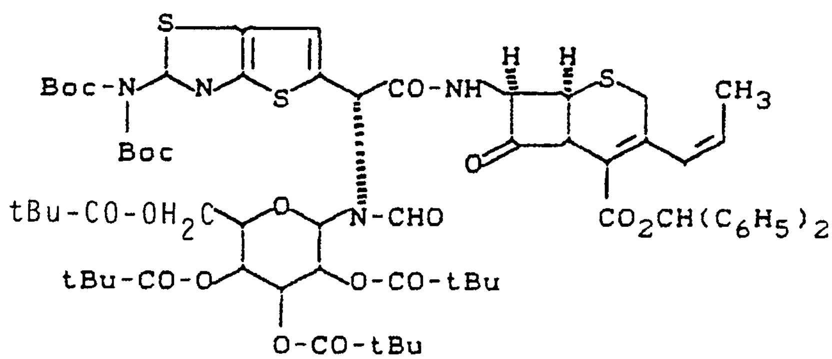

Zu einer Lösung von 1,0 g (2,6 mmol) der Verbindung aus Beispiel 5, 1,29 g (2,5 mmol) Tetra-0-pivaloyl-galacto-sylamin, 0,92 g (2,2 mmol) der Verbindung aus Beispiel 11, 1,0 ml (26,5 mmol) Ameisensäure in 30 ml wasserfreiem Tetrahydrofuran gibt man 0,6 ml (2,0 mmol) Titan(IV)isopropylat und rührt 5 d bei Raumtemperatur. Nach dem Abziehen des Lösemittels wird an Kieselgel mit Toluol/Essigester (3:1) chromatographiert.

Ausbeute: 0,31 g (10% der Theorie)

$R_f$ = 0,28 (Kieselgel 60, F 254, Merck-Nr. 5549, Toluol/Essigester 3:1)

Beispiel 13

7-[2-(2-Amino-5-thieno[2,3-d]thiazolyl)-2-formylamino-acetylamino]-3-[(Z)-1-propenyl]-(6R, 7R)-3-cephem-4-carbonsäure (Trifluoracetat)

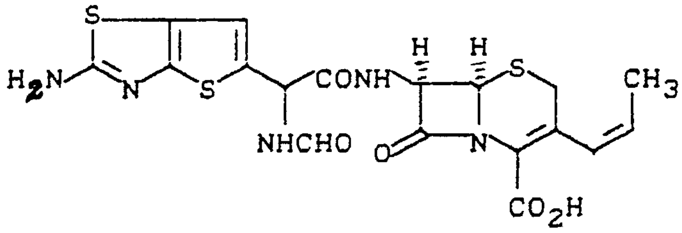

x $CF_3CO_2H$

250 mg (0,24 mmol) der Verbindung aus Beispiel 9 werden 1 h bei Raumtemperatur mit einer Mischung aus 1 ml Anisol und 10 ml Trifluoressigsäure behandelt. Nach dem Einengen wird das Produkt mit Ether ausgefällt, abgesaugt, mit Ether gewaschen und im Vakuum getrocknet. Ausbeute: 110 mg (76% der Theorie)

Beispiel 13a (allgemeine Formel (I))

7-[(2S)-2-(2-Amino-5-thieno[2,3-d]thiazolyl )-2-amino-acetylaminol-3-[(Z)-1-propenyl]-(6R, 7R)-3-cephem-4-carbonsäure

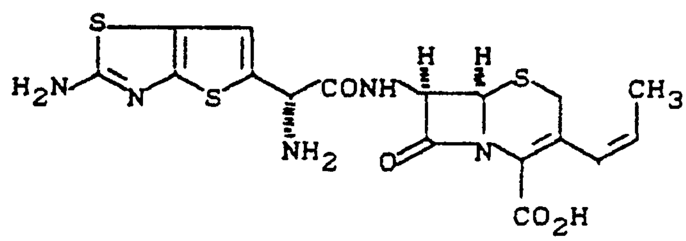

107 mg (0,18 mmol) der Verbindung aus Beispiel 13 werden in 10 ml Methanol gelöst. Man fügt 1 ml konzentrierte Salzsäure zu und läßt 48 h bei Raumtemperatur stehen. Anschließend wird mit 200 ml Wasser verdünnt und mit Natriumacetat auf pH 5 gestellt. Chromatographie an HP-20 mit Wasser/Acetonitril liefert das reine D-Isomer.

Ausbeute: 7 mg (9% der Theorie)

$^1$H-NMR (DCOOD): $\delta$ = 1,66 (dd, J = 7 Hz, J = 1 Hz, 3H, $CH_3$); 3.44 und 3,54 (2d, J = 18 Hz, je 1H, 2-H); 5,28 (d, J = 6 Hz, 1H, 6-H); 5,84 (d, J = 6 Hz, 1H, 7-H): 5,80 (dq, J = 11 Hz, J = 7 Hz, 1H, 3'-H): 5,93 (s, 1H, N-CH-C0); 6,23 (d, J = 11 Hz, 1H, 3'-H); 7,64 (s, 1H, Ar-H).

## Ansprüche

**1.** Verbindungen der Formel (I),

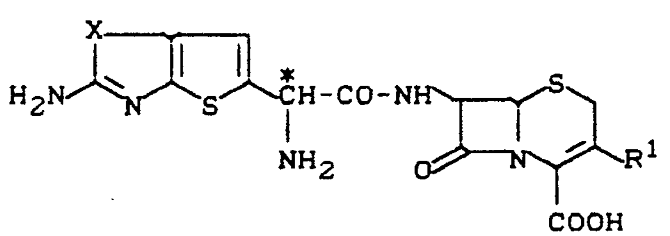

(I)

in welcher

$R^1$ - für Halogen oder

- für geradkettiges oder verzweigtes Alkyl, Alkylthio oder Alkenyl mit bis zu 10 Kohlenstoffatomen steht, die gegebenenfalls durch Halogen, geradkettiges oder verzweigtes Alkoxy mit bis zu 8 Kohlenstoffatomen oder durch einen 5-gliedrigen ungesättigten Heterocyclus mit bis zu 4 Heteroatomen aus der Reihe Stickstoff oder Schwefel substituiert sind, der seinerseits durch geradkettiges oder verzweigtes Alkyl oder Alkylaminodialkyl mit bis zu 6 Kohlenstoffatomen substituiert sein kann, oder die gegebenenfalls durch eine Gruppe der Formel $-OCOR^2$ substituiert sind,

worin

$R^2$ - Amino oder geradkettiges oder verzweigtes Alkyl mit bis zu 6 Kohlenstoffatomen bedeutet,
- für geradkettiges oder verzweigtes Alkoxy mit bis zu 8 Kohlenstoffatomen steht,
- für eine Gruppe der Formel $-CH_2-S-R^3$ steht,

worin

$R^3$ - einen 5-gliedrigen ungesättigten Heterocyclus mit bis zu 4 Heteroatomen aus der Reihe Stickstoff oder Schwefel bedeutet, der gegebenenfalls durch geradkettiges oder verzweigtes Alkyl oder Alkylaminodialkyl mit bis zu 6 Kohlenstoffatomen substituiert ist,

x - für Sauerstoff, Schwefel oder für den Rest -NH steht

und deren physiologisch unbedenklichen Salze.

**2.** Verbindungen nach Anspruch 1,
in welchen

$R^1$ - für Fluor, Chlor oder Brom steht, oder

- für geradkettiges oder verzweigtes Alkyl, Alkylthio oder Alkenyl mit bis zu 8 Kohlen stoffatomen steht, die gegebenenfalls durch Fluor, Chlor, Brom oder durch geradkettiges oder verzweigtes Alkoxy mit bis zu 6 Kohlenstoffatomen oder durch Tetrazolyl, Thiazolyl, Triazolyl oder Thiadiazolyl substituiert sind, welche ihrerseits durch geradkettiges oder verzweigtes Alkyl oder Alkylaminodialkyl mit bis zu 4 Kohlenstoffatomen substituiert sein können, oder
- die gegebenenfalls durch eine Gruppe der Formel $-OCOR^2$ substituiert sind,

worin

$R^2$ - Amino oder geradkettiges oder verzweigtes Alkyl mit bis zu 4 Kohlenstoffatomen bedeutet,
- für geradkettiges oder verzweigtes Alkoxy mit bis zu 6 Kohlenstoffatomen steht,

x - für Sauerstoff oder Schwefel steht, und deren physiologisch unbedenklichen Salze.

**3.** Verbindungen nach Anspruch 1,
in welchen

$R^1$ - für Chlor oder geradkettiges oder verzweigtes Alkyl oder Alkenyl mit bis zu 6 Kohlenstoff atomen steht, die gegebenenfalls durch Fluor, Chlor oder geradkettiges oder verzweigtes Alkoxy mit bis zu 4 Kohlenstoffatomen substituiert sind, oder
- für geradkettiges oder verzweigtes Alkoxy mit bis zu 4 Kohlenstoffatomen steht,

x - für Schwefel steht und deren physiologisch unbedenklichen Salze.

**4.** Verfahren zur Herstellung der Verbindungen der allgemeinen Formel (I),

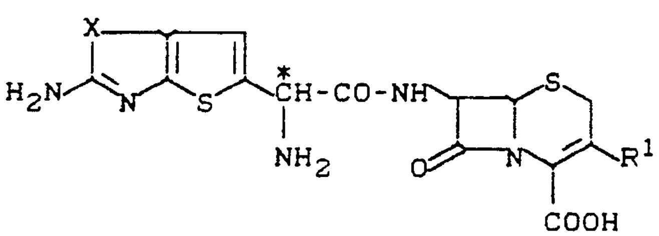

(I)

in welcher
$R^1$ und X die in Anspruch 1 angegebenen Bedeutungen haben,

dadurch gekennzeichnet, daß man entweder

[A] in einer 4-Komponentenreaktion Verbindungen der allgemeinen Formeln (II), (III) und (IV),

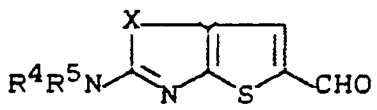,
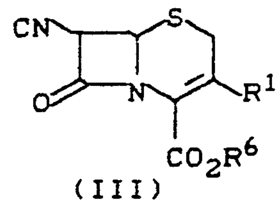
und
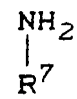

(II) (III) (IV)

in welchen

$R^1$ und X die oben angegebene Bedeutung haben,

$R^4$ und $R^5$ gleich oder verschiedan sind und für eine Aminoschutzgruppe, beispielsweise für tert.-Butyloxycarbonyl (Boc), stehen,

$R^6$ für eine Carboxyschutzgruppe steht,

und

$R^7$ - für einen leicht abspaltbaren, benzylisch oder glykosidisch gebundenen Rest, wie beispielsweise 4,4'-Dimethoxybenzhydryl oder 2,4-Dimethoxybenzyl oder Tetra-0-pivaloylgalactosyl steht, oder

- für einen benzylisch, chiralen Rest, wie beispielsweise ∝-Ferrocenyl-($C_2$-$C_6$)-alkylamino steht,

in Anwesenheit einer Carbonsäure der Formel (V),

$R^8$-COOH (V)

in welcher

$R^8$ - Wasserstoff, geradkettiges oder verzweigtes Alkyl mit bis zu 6 Kohlenstoffatomen oder einen Rest der Formel

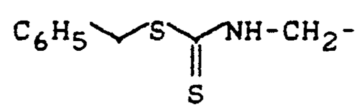

bedeutet,

in inerten Lösemitteln, gegebenenfalls in Anwesenheit eines Katalysators zu Verbindungen der allgemeinen Formel (Ia),

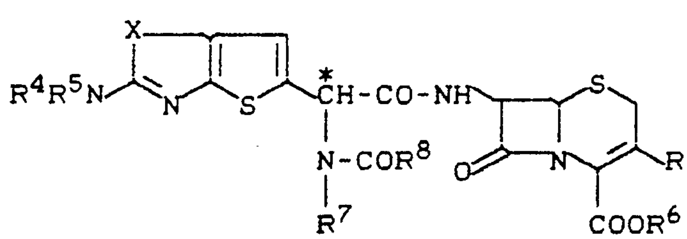

(Ia)

in welcher

$R^1$, $R^4$, $R^5$, $R^6$, $R^7$, $R^8$ und X die oben angegebene Bedeutung haben,
umsetzt, anschließend die Schutzgruppen $R^4$, $R^5$, $R^6$ und die Reste $R^7$ und -$COR^8$ mit Säuren abspaltet,
oder daß man

[B] Carbonsäuren der allgemeinen Formel (VI),

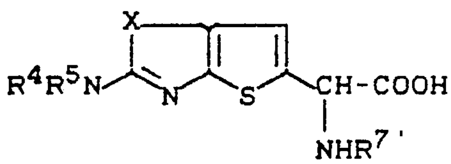

(VI)

in welcher
$R^3$, $R^4$ und x die oben angegebene Bedeutung haben
und

$R^{7'}$ - die oben angegebene Bedeutung von $R^7$ hat und mit dieser gleich oder verschieden ist, oder

- für eine Aminoschutzgruppe steht,

nach Aktivierung der Carboxylgruppe nach ublicher Methode, mit einer ß-Lactam-Verbindung der allgemeinen Formel (VII),

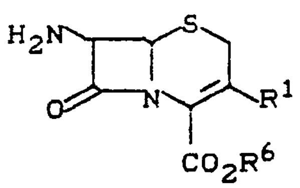

(VII)

in welcher
$R^1$ und $R^6$ die oben angegebene Bedeutung haben,
in inerten Lösemitteln umsetzt und im letzten Schritt gegebenenfalls die Schutzgruppen $R^4$, $R^5$, $R^6$ und $R^{7'}$ abspaltet.

**5.** Verwendung von Verbindungen gemäß den Ansprüchen 1 bis 3 zur Herstellung von Arzneimitteln.

Europäisches Patentamt

# EUROPÄISCHER RECHERCHENBERICHT

Nummer der Anmeldung

EP 90121995.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | US - A - 4 839 351 (T. NISHIMURA et al.) * Ansprüche 1,7; Spalten 1, 13,27,28,46 * | 1-5 | C 07 D 519/00 A 61 K 31/545// |
| A | US - A - 4 826 834 (Y. YOSHIMURA et al.) * Ansprüche 1,12; Spalten 1, 12,27,48 * | 1-5 | |
| A | US - 4 748 172 (A.S. KATNER) * Ansprüche 1,2; Spalten 1,2, 12,13 * | 1-5 | |
| A | EP - A2 - 0 286 144 (F. HOFFMANN-LA ROCHE & CO. AKTIENGESELLSCHAFT) * Ansprüche 1,28,29; Seite 6 * | 1-5 | |

RECHERCHIERTE SACHGEBIETE (Int Cl 5)

C 07 D 519/00

Der vorliegende Recherchenbericht wurde für alle Patentansprüche erstellt

| Recherchenort | Abschlußdatum der Recherche | Prüfer |
|---|---|---|
| WIEN | 27-02-1991 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument